# EUROPEAN PATENT SPECIFICATION

(11) **EP 2 053 993 B1**
(45) Date of publication and mention of the grant of the patent: **09.08.2017**
(21) Application number: 07805760.1
(22) Date of filing: 03.08.2007
(51) Int. Cl.: A61F 2/30, A61F 2/40

(54) **SHOULDER PROSTHESIS**
SCHULTERPROTHESE
PROTHÈSE D'ÉPAULE

(30) Priority: 03.08.2006 IT MI20061568
(43) Date of publication of application: 06.05.2009
(73) Proprietor: Plustek S.R.L., 20135 Milano (IT)
(72) Inventor: GIULIANI, Alessio, 65125 Pescara (IT); BOCENTI, Valter, 29100 Piacenza (IT); BRAMBILLA, Elia, 20062 Cassano d'Adda (IT); SALA, Giuseppe, 20033 Desio (IT); GUERRA, Paolo, 20145 Milano (IT); RIZZI, Luca, 20144 Milano (IT)
(74) Representative: Postiglione, Ferruccio
(86) International application number: PCT/IT2007/000567
(87) International publication number: WO 2008/015724

(56) References cited:
- EP-A- 1 059 071
- EP-A- 1 472 999
- WO-A-94/15551
- WO-A-03/065939
- WO-A1-98/15241
- FR-A- 2 686 503
- FR-A- 2 704 747
- FR-A- 2 780 635
- GB-A- 1 292 561
- JP-A- 2006 174 946
- US-A- 5 702 486
- US-A1- 2004 064 187
- US-A1- 2004 220 673
- US-A1- 2005 177 241

## Description

The present invention relates to shoulder prosthesis and in particular to nodular type shoulder prosthesis, suitable for changing from a direct configuration to an inverted configuration. The direct configuration consists of a shoulder prosthesis wherein a head is associated with the main body of the prosthesis to replace the humeral head, suitable for being coupled with the glenoid, while the inverted configuration consists of a shoulder prosthesis wherein a ball is associated with the glenoid suitable for being coupled with a cup associated with the humerus.

Shoulder prostheses according to prior art comprise a main prosthesis body adapted for insertion into the humerus and a ball and socket type coupling to permit relative movements between humerus and glenoid. Such solutions are known from US 5702486, US 2004/064187 A1, GB 1292561 A, WO 98/15241 A1, JP 2006 174946 A.

However, said prostheses present numerous drawbacks. For example the prostheses according to prior art do not permit complete recovery of the biomechanical articular function after surgery. In fact the loss of recovery of this function is mainly caused by incorrect suturing of the tuberosities of the humeral head. Lack of functional recovery often makes the prosthesis painful for the patient, and in any case, it limits the shoulder movement of the patient to a large extent.

Till now, therefore, current shoulder prostheses often have not provided satisfactory recovery of the articular function.

Furthermore, the prostheses according to prior art often result as being excessively invasive and therefore, in order to permit their insertion, a large amount of bone must be removed, thus causing problems for prosthesis integration and

Furthermore, the prostheses according to prior art often do not permit the correction of certain important geometric parameters such as the distance between the acromion and the head and the version angle of the head, except by extremely invasive operations requiring the explant of the prosthesis and its total or partial replacement. These operations require interventions that are extremely invasive and painful for the patient, comprising the filing of the neck of the humeral head, with the consequential ablation of a considerable amount of bone.

The prostheses according to prior art do not permit safe and not particularly invasive attachment to the bone especially in relation to the glenoid. In fact it is often necessary to use numerous very invasive screws which not only complicate the prosthesis implant surgical operation considerably, but also contribute towards weakening the resistant sections of bone that are already particularly reduced from an anatomical point of view.

For anchoring to the bone, the prostheses according to prior art use pass-through screws that perforate the cortex and penetrate the spongy tissue.

The use of the drilling method complicates the surgical insertion stage also because incorrect hole drilling can lead to bone fractures. Furthermore, any error in prosthesis positioning also involves the drilling of an invasive hole which can be damaging to bone resistance.

The object of the present invention is to provide a shoulder prosthesis that overcomes the drawbacks described in relation to prior art.

These drawbacks are overcome by shoulder prosthesis according to independent claim 1.

Other embodiments of the prosthesis according to the invention are described in the following dependent claims.

Further characteristics and advantages of the present invention will be made more clear in the description below and the preferred non-limiting embodiments, wherein:

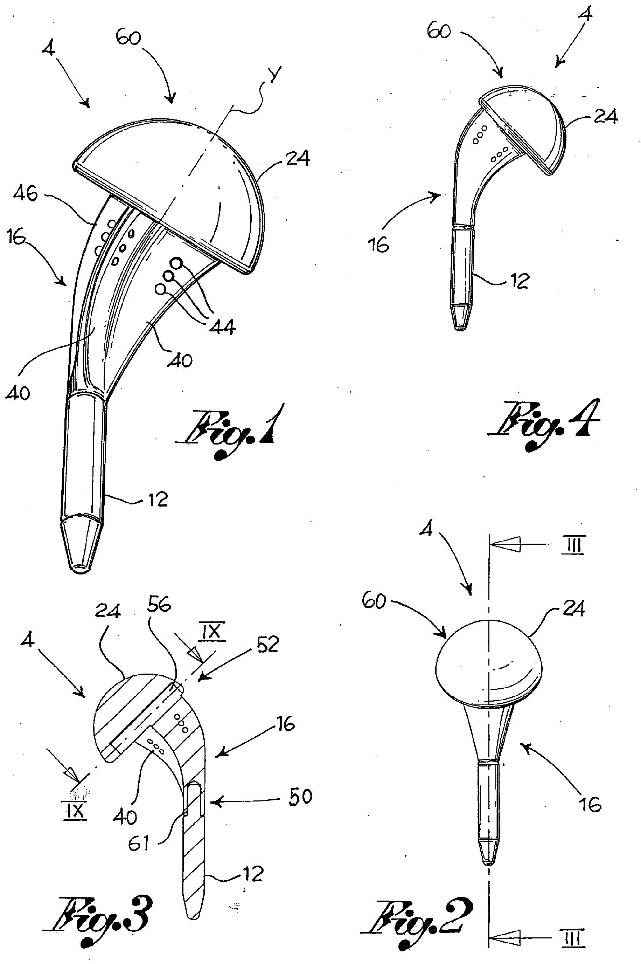

Figure 1 shows a perspective view of a shoulder prosthesis according to one embodiment of the present invention;
Figure 2 shows a front view of the prosthesis in figure 1;
Figure 3 shows a cross-section view of the prosthesis in figure 1, along the section line III-III in figure 2;
Figure 4 shows a side view of the prosthesis in figure 1;

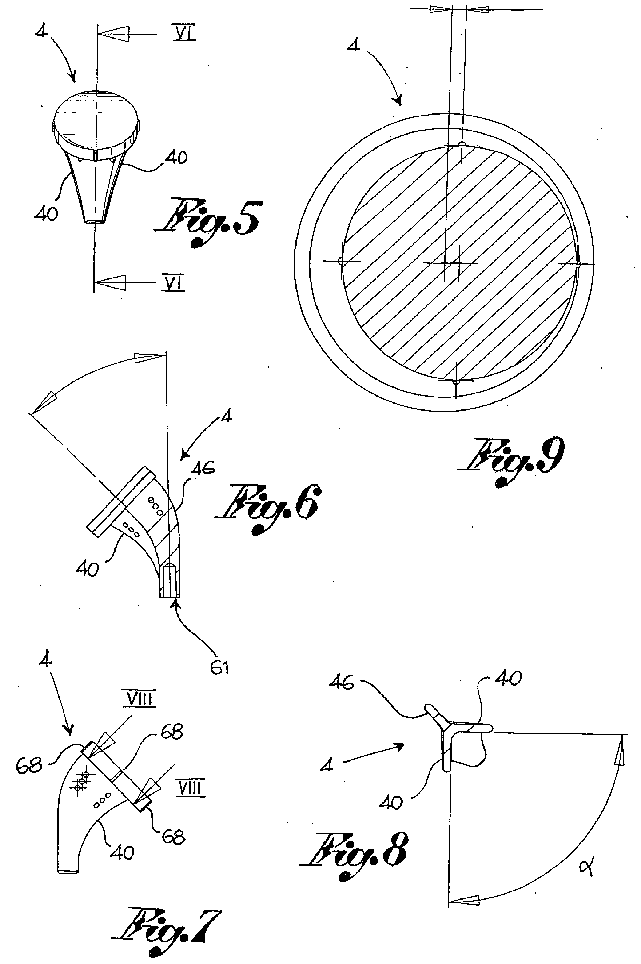

Figure 5 shows a front view of a detail of a prosthesis according to one embodiment of the present invention;
Figure 6 shows a cross-section view of the detail in figure 5, along the section line VI-VI in figure 5;
Figure 7 shows a side view of the detail in figure 5;
Figure 8 shows a cross-section view of the detail in figure 5, along the section line VIII-VIII in figure 5;
Figure 9 shows a cross-section view of the prosthesis in figure 3, along the section line IX-IX in figure 3;

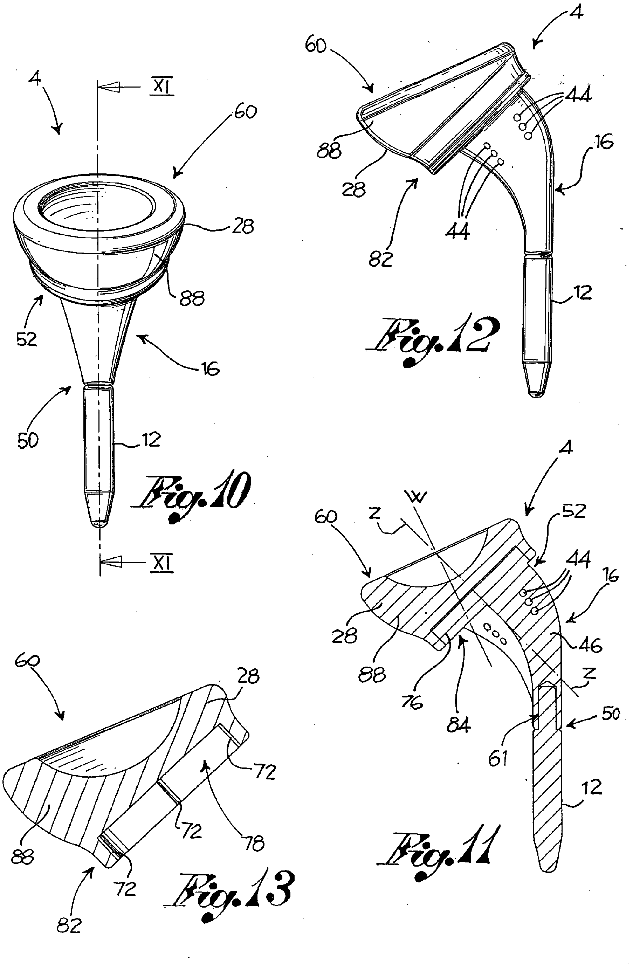

Figure 10 shows a perspective view of a shoulder prosthesis according to a further embodiment of the present invention;
Figure 11 shows a cross-section view in relation to the section line XI-XI in figure 10;
Figure 12 shows a side view of the prosthesis in figure 10;
Figure 13 shows a cross-section view of a detail of the prosthesis in figure 10;

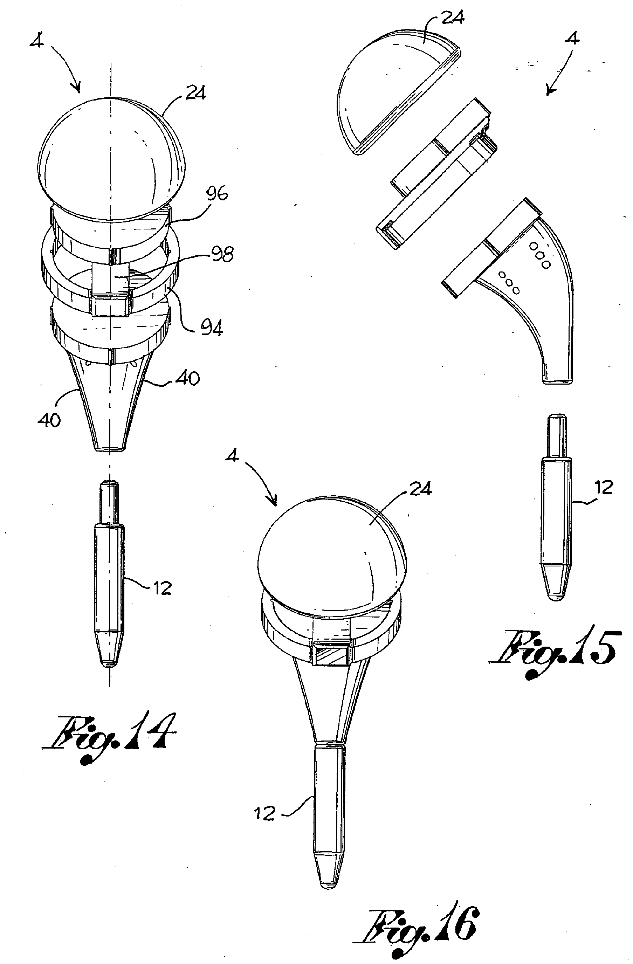

Figure 14 shows a view in separate parts of a shoulder prosthesis according to a further embodiment of the present invention;
Figure 15 shows a side view of the prosthesis in figure 14;
Figure 16 shows a front view of the prosthesis in figure 14 in an assembled configuration;

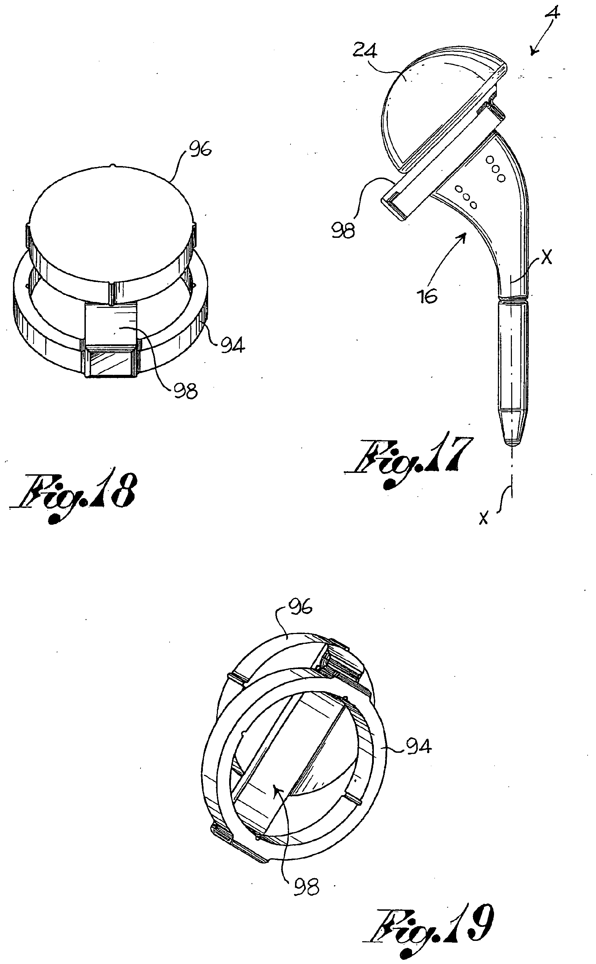

Figure 17 shows a side view of the prosthesis in figure 16;
Figures 18 and 19 show views from different angles of a detail of the prosthesis in figure 14;

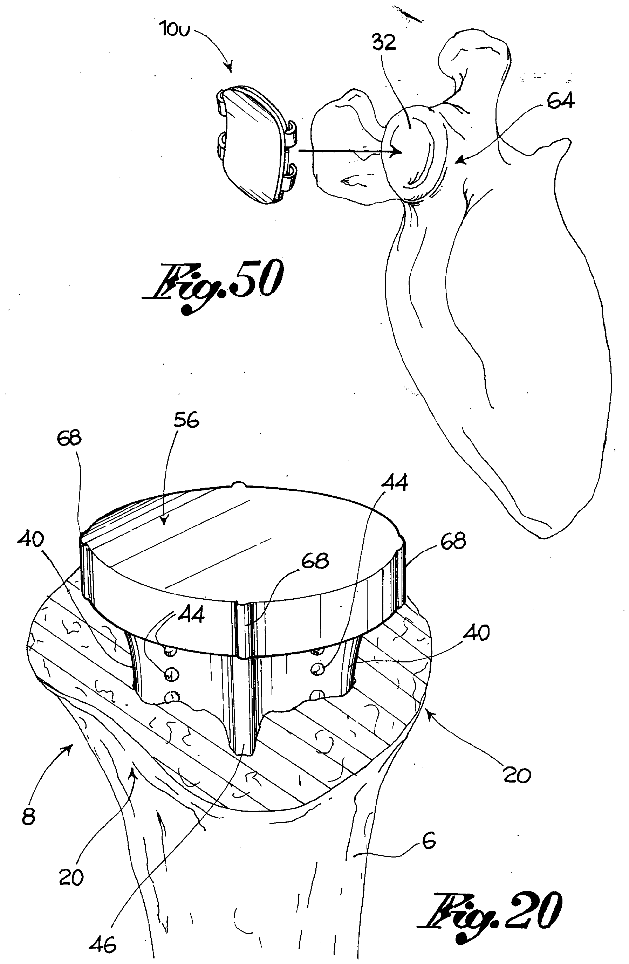

Figure 20 shows a perspective view of a prosthesis according to the invention in a configuration of the insertion into a humerus;

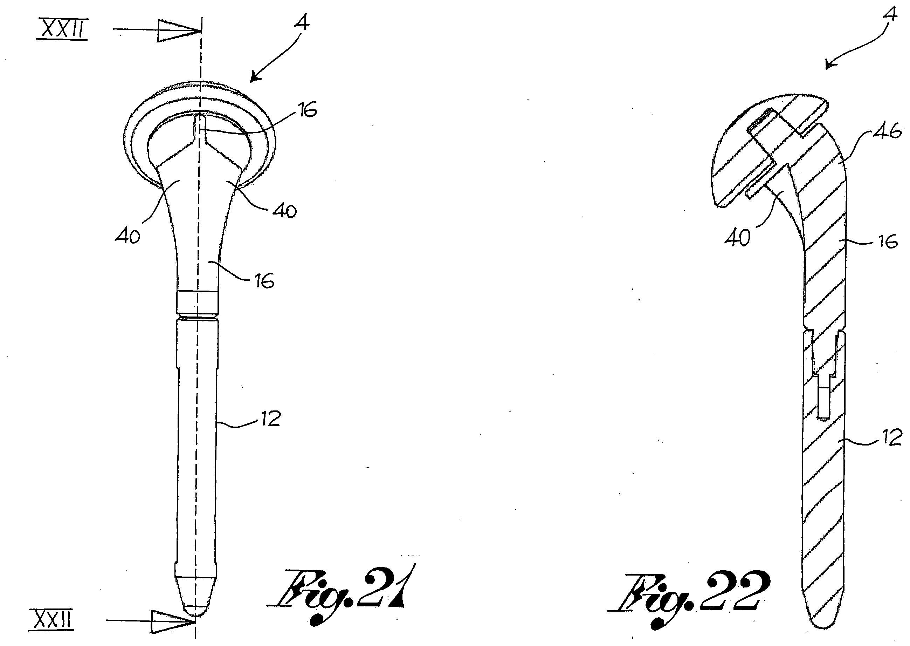

Figure 21 shows a front view of a shoulder prosthesis according to a further embodiment of the present invention;
Figure 22 shows a cross-section view of the prosthesis in figure 21, along the section line XXII-XXII in figure 21;

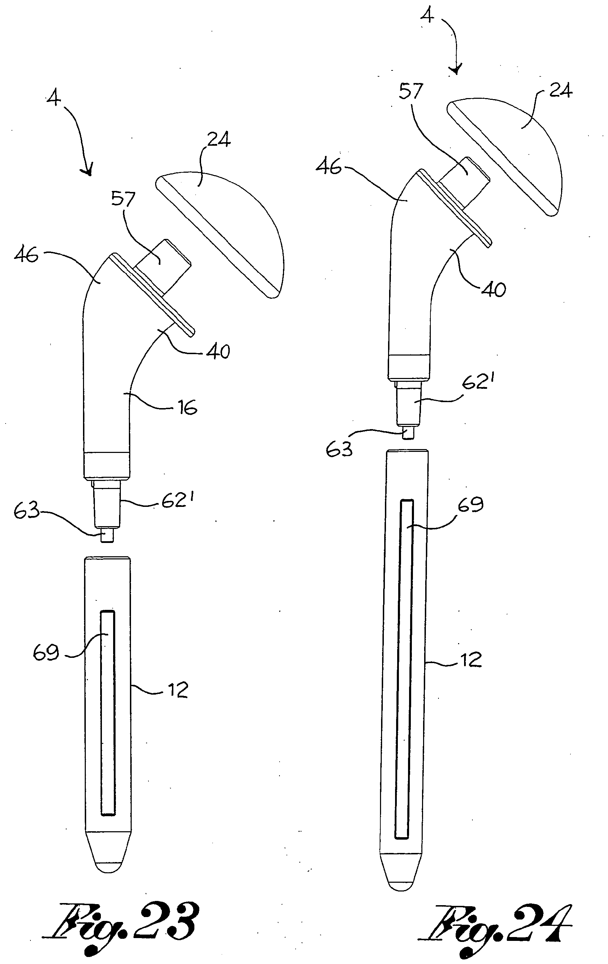

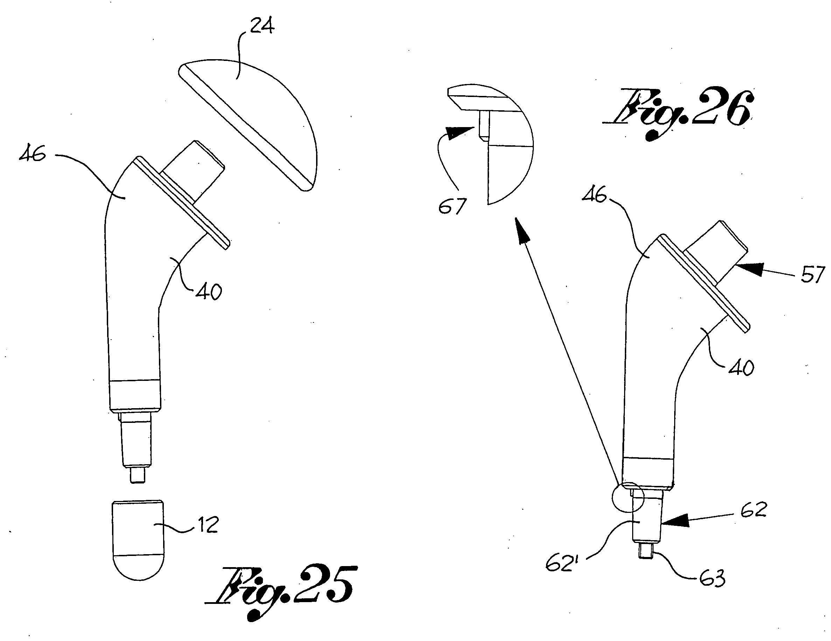

Figures 23-25 show views in separate parts of the prosthesis in figure 22, according to further variants of the embodiment;
Figure 26 shows a side view of a detail of the prosthesis in figure 21;

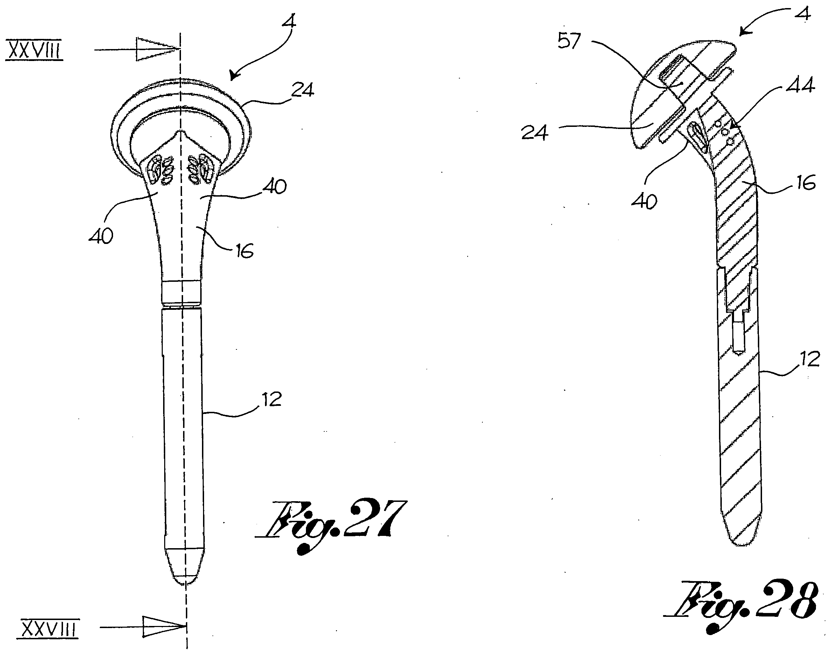

Figure 27 shows a front view of a shoulder prosthesis according to a further embodiment of the present invention;
Figure 28 shows a cross-section view of the prosthesis in figure 27, along the section line XXVIII-XXVIII in figure 27;

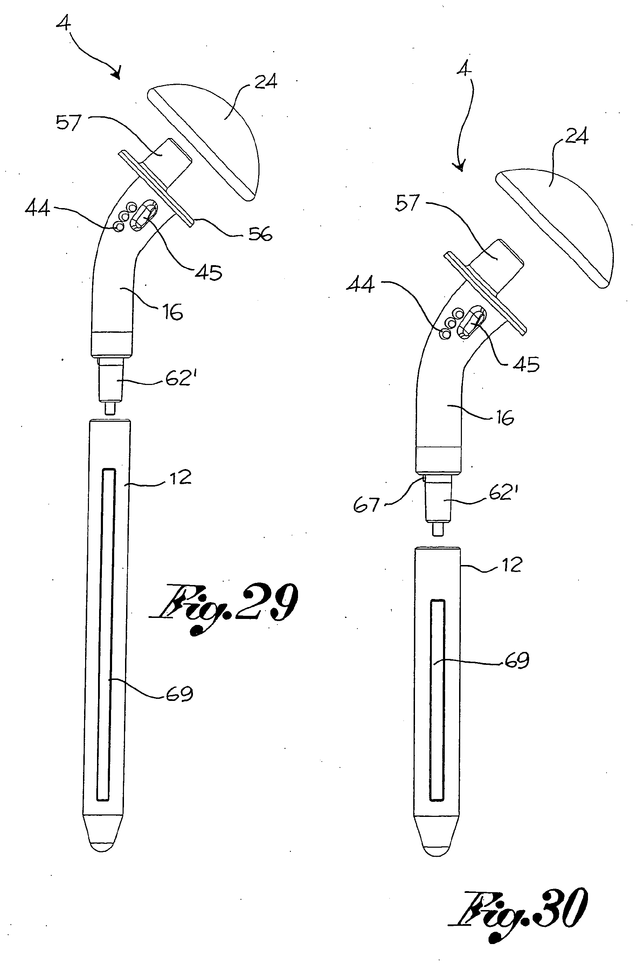

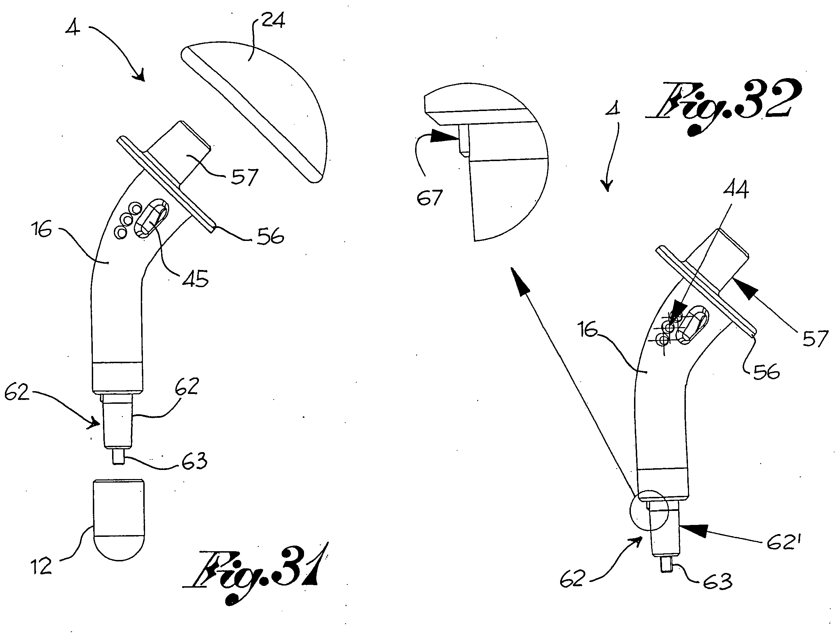

Figures 29-31 show in separate parts of the prosthesis in figure 27, according to further variants of the embodiment;
Figure 32 shows a side view of a detail of the prosthesis in figure 27;

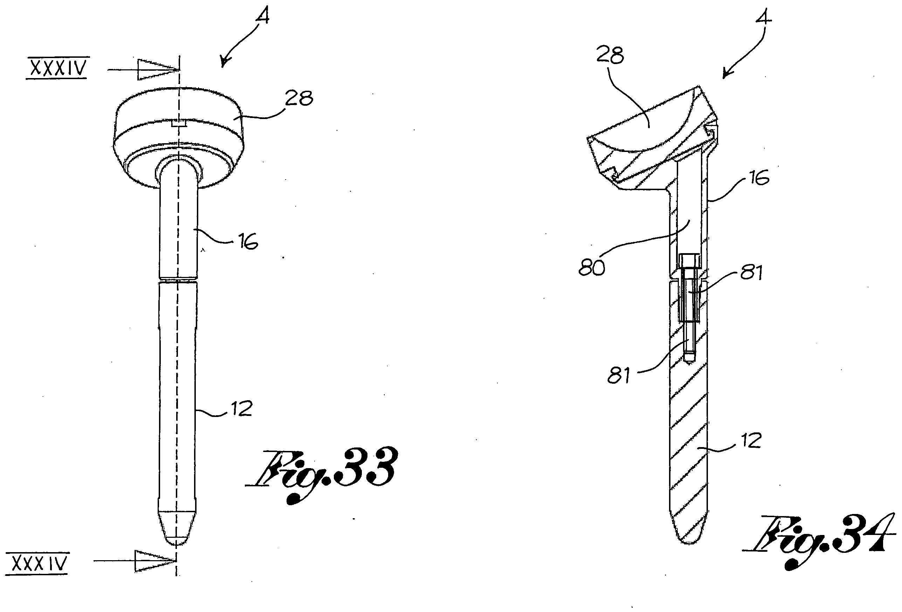

Figure 33 shows a front view of a shoulder prosthesis;
Figure 34 shows a cross-section view of the prosthesis in figure 33, along the section line XXXIV-XXXIV in figure 33;

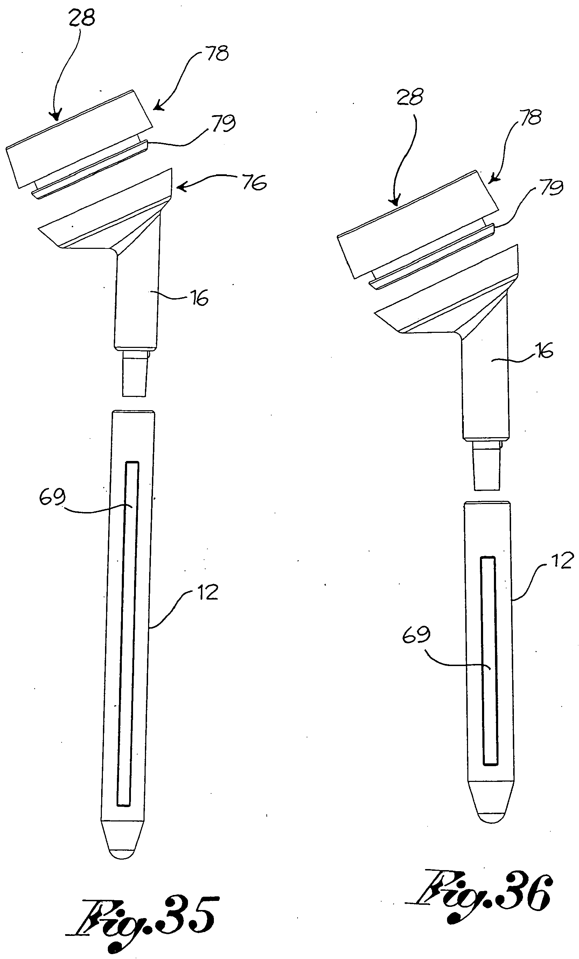

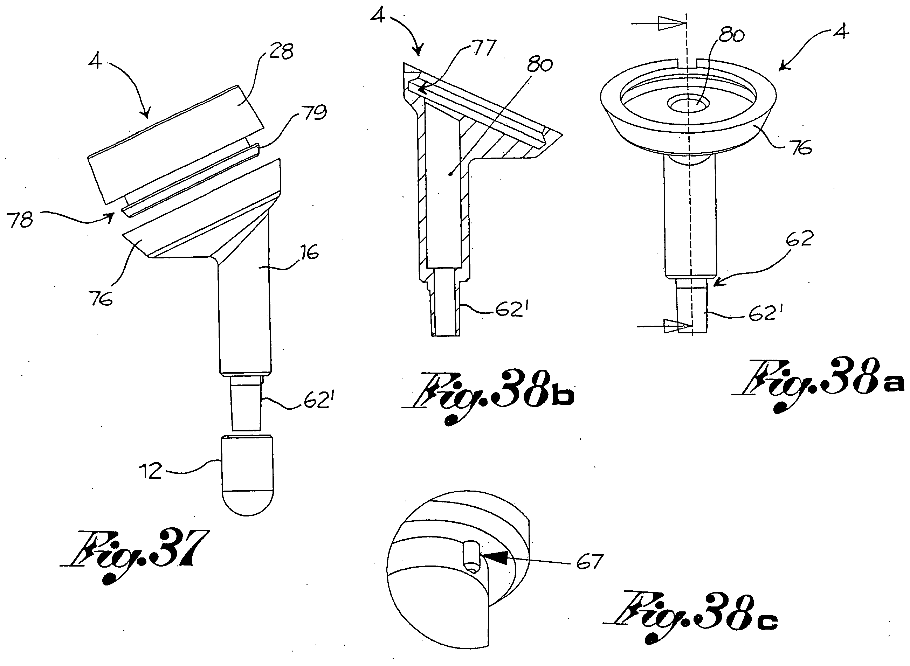

Figures 35-37 show in separate parts of the prosthesis in figure 33;
Figures 38a and 38b show side views and in cross section of a detail of the prosthesis in figure 33;

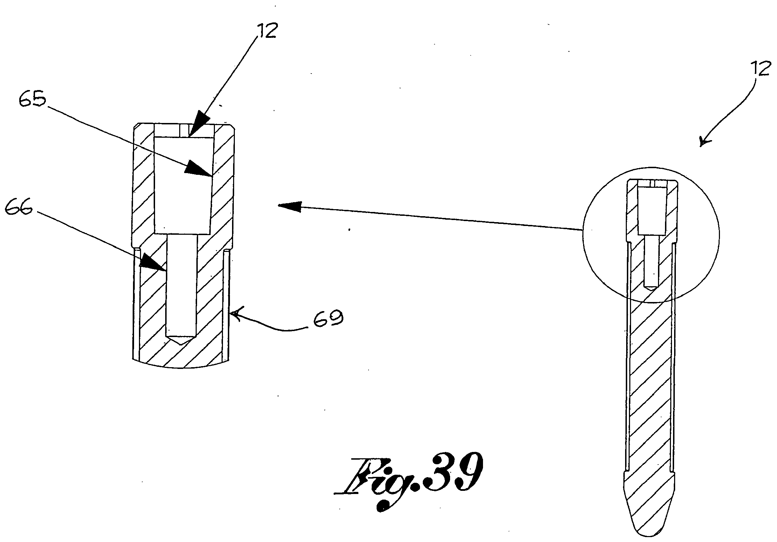

Figure 39 shows a cross-section view of an enlarged detail of a component of a prosthesis according to the present invention;

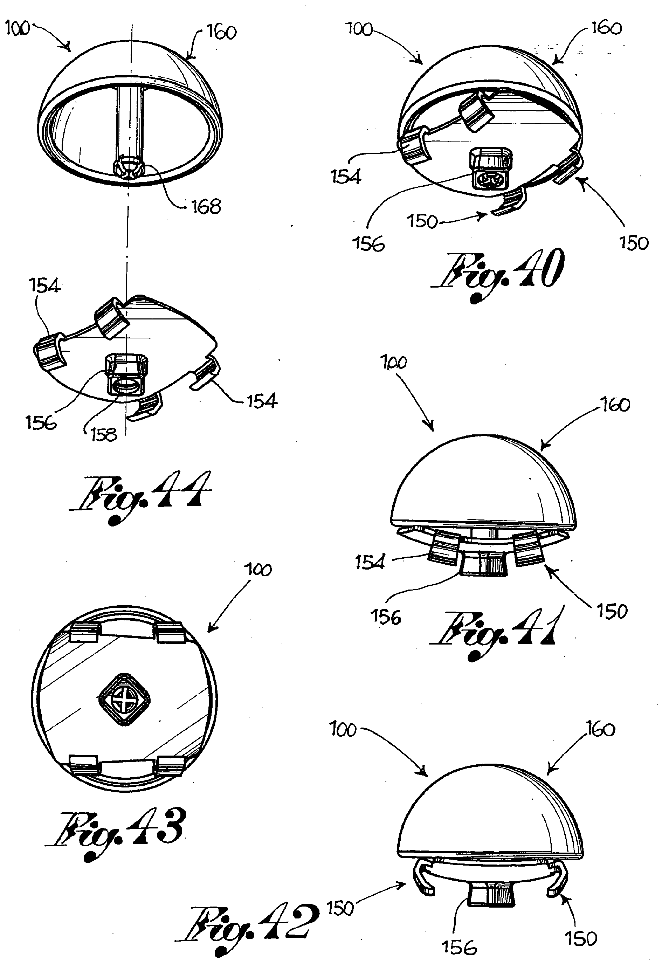

Figure 40 shows a perspective view in an assembled configuration of a shoulder prosthesis according to a further embodiment of the present invention;
Figures 41 and 42 show side views from different ang les of the prosthesis in figure 21;
Figure 43 shows a plane view from below of the prosthesis in figure 40;
Figure 44 shows a perspective view in separate parts of the prosthesis in figure 40;

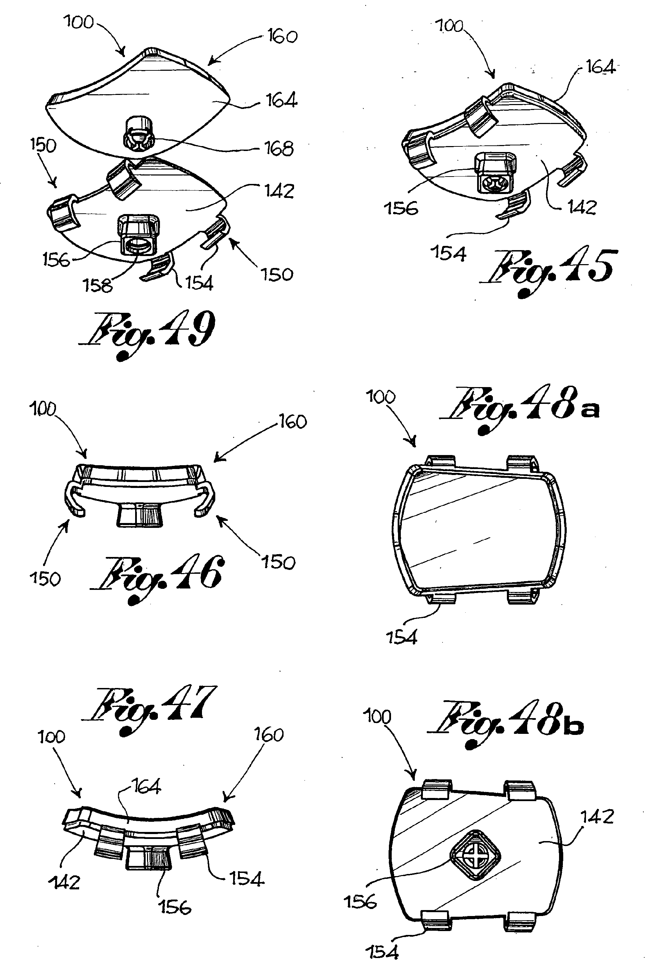

Figure 45 shows a perspective view of a shoulder prosthesis according to a further embodiment of the present invention;
Figures 46 and 47 show side views from different angles of the prosthesis in figure 45;
Figures 48A and 48B show plane views seen from above and below of the prosthesis in figure 45 respectively;
Figure 49 shows a perspective view in separate parts of the prosthesis in figure 45;
Figure 50 shows a perspective view of a prosthesis according to the present invention in a configuration assembled on a scapula (during preparation);

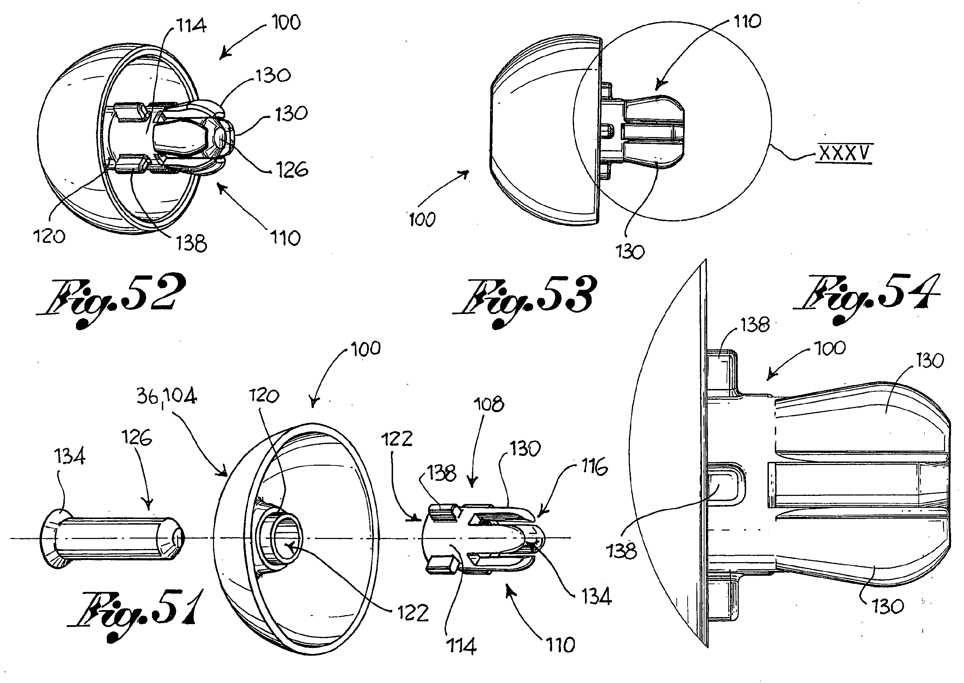

Figure 51 shows a perspective view in separate parts of a shoulder prosthesis according to a further embodiment of the present invention;
Figure 52 shows a perspective view in an assembled configuration of the prosthesis in figure 51;
Figure 53 shows a side view of the prosthesis in figure 52;
Figure 54 shows an enlarged detail of the prosthesis in figure 53.

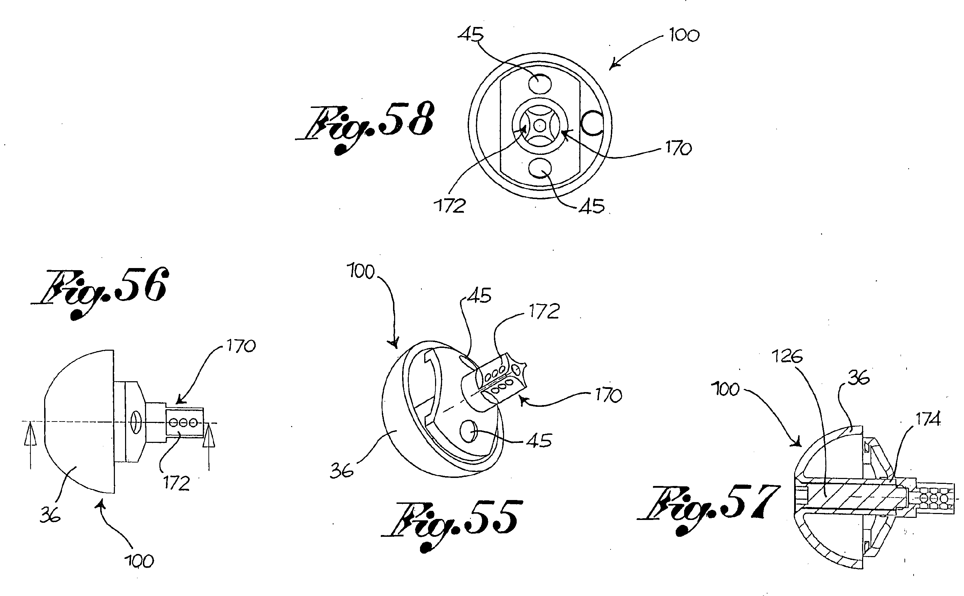

Figures 55-58 show perspective views, side views and views in cross section of shoulder prosthesis according to a further embodiment of the present invention.

The common elements or parts of elements present in the embodiments described hereafter will be identified by the same numerals.

In reference to the aforesaid figures, according to one embodiment of the present invention throughout, the numeral 4 indicates a shoulder prosthesis suitable for insertion in a humerus 6, preferably positioned on the head 8 of said humerus 6.

According to one embodiment, the prosthesis 4 comprises a stem 12 suitable for insertion into the humerus to attach the prosthesis 4 on the humerus 6, the stem identifying a direction of insertion X.

The prosthesis 4, according to one embodiment, comprises a main body of the prosthesis 16 or metaphysary component, attachable to said stem 12, and adapted for insertion at least partially into the humerus positioned on the humeral head 8 having a pair of tuberosities 20.

Preferably, the stem 12 has an axis of symmetry in relation to the insertion direction X, in a manner to form a revolving surface adapted to rotation inside said humerus to permit the correct angular positioning of the prosthesis 4 in the humerus.

The main body of the prosthesis 16 extends according to a prevalent extension Y set at an angle in relation to the insertion direction X in a manner to follow the anatomical angle of the humeral head 8. Preferably said prevalent extension Y forms a curved line.

The main body of the prosthesis 16 is adapted to being associated with a spherical head 24 or with a cup 28 in a manner to interact with a glenoid cavity 32, if necessary covered with a relative plate 142, or with a glenosphere 36, respectively.

The main body of the prosthesis 16 comprises support fins 40 that together form, in relation to a plane perpendicular to said prevalent extension Y, a flare angle α between 60 and 120 degrees in a manner to be abutted by said tuberosities 20 of the humerus, by the internal part of the bone, in an insertion configuration of the prosthesis in the humerus.

Preferably, said fins 40 together form, in relation to a plane perpendicular to said set at an angle direction, a flare angle α between 80 and 100 degrees.

Even more preferably, said fins 40 together form, in relation to a plane perpendicular to said set at an angle direction, a flare angle α equal to 90 degrees.

Advantageously, the support fins 40 present at least one hole 44 adapted to allowing the passage of a suture for the resuturing of said tuberosities 20.

Preferably, said fins 40 present three holes 44 arranged along the prevalent extension Y of the main body of the prosthesis 12, said holes 44 being adapted to allowing the passage of a suture for the resuturing of said tuberosities 20.

According to a possible embodiment, said fins 40 present slots 45 adapted to allowing an easier passage of the suture for the resuturing of said tuberosities 20 as well as to permit the insertion of the bone fragments to favour the osteointegration of the prosthesis 4.

The support fins 40 have a blade-like configuration and extend from said prevalent extension Y, in such a manner that the main body of the prosthesis 16 assumes a general section that is bilobate.

The main body of the prosthesis 16 comprises a third fin 46, positioned symmetrically in relation to said support fins 44, said third fin extending from the opposite side to the support fins 44 in relation to the prevalent extension Y, in such a manner that the main body of the prosthesis 16 assumes a general "Y" shaped cross-section.

According to one embodiment, at least one of said fins 40, 44 extends, in relation to the axis of the prevalent extension Y, in an increasing distance, moving from a first end 50 of the main body of the prosthesis 16, from the attachable stem 12, to a second end 52 of the main body of the prosthesis 16, opposite to said first end 50.

Preferably, the configuration of the main body of the prosthesis 16 comprising two fins 40 and a third fin 46 is adapted to being used for shoulder arthrosis pathologies; a configuration comprising two fins 40, without the third fin 46, is preferably used in the case of fractures of the humeral head 8.

According to one embodiment of the present invention, said fins 40,44 opposite the stem 12, are operatively connected to a plate or disc 56 attachable by means of form coupling to a coupling portion 60 adapted to creating kinematic coupling with the glenoid.

According to a further embodiment of the present invention, said disc 56 is associated with a first cone 57, preferably of the 'morse' type adapted to favouring the fixing action of said coupling portion 60 to the main body of the prosthesis 16, by means of form coupling. The coupling portion 60, in the form of a spherical head 24 for example, will be provided with a housing having the counter form of the first cone 57 in a manner to ensure the secure blocking of the spherical head 24 on the main body of the prosthesis 16.

Preferably, the connection between the main body of the prosthesis 16 and the stem 12, positioned at said first end 50 of the main body of the prosthesis 16, is achieved by means of form coupling.

For example, the stem 12 comprises a threaded pin, which is screwed directly into a relative nut screw 61 of the main body of the prosthesis 16. Preferably, the main body of the prosthesis 16, positioned at said first end 50, comprises a connecting protuberance 62, presenting a second cone 62', preferably of the 'morse' type, and a threaded portion 63. The stem 12 possesses a conical seat 65 adapted to blocking inside the second cone 62' and a threaded hole 66. According to a possible embodiment, said threaded hole 66 is adapted to being screwed into the threaded portion 63 of the connecting protuberance 62.

Preferably, the connecting protuberance 62 comprises a rotation-proof ridge 67 adapted to preventing undesirable rotation between the stem 12 and the main body of the prosthesis 16.

According to a possible embodiment, the stem 12 presents at least one groove 69, being parallel to the direction of the prevalent extension X-X for example, in a manner to create a rotation proof action of stem 12 following the new growth of the bone; in other words, as the bone grows it will penetrate the groove 69 to form an undercut.

According to one embodiment, the coupling portion 60 comprises a spherical head 24, on the opposite side of the main body of the prosthesis 16, adapted to creating kinematic coupling with a glenoid 64 of the attachable scapula.

According to one embodiment, said disc 56 presents at least one ribbing 68 positioned on an axial rim and said coupling portion presents at least one groove 72 having the counter form of said ribbing 68.

Preferably, the disc 56 comprises four ribbings 68 in step formation, preferably perpendicular to each other, and in turn, the coupling portion 60 presents four grooves 72 set perpendicular to one another to permit coupling with said ribbing 68.

Preferably, the area of the section of the fins 40,44 in relation to a plane perpendicular to said prevalent extension Y does not exceed 85% of the total area of a disc associated with the main body of the prosthesis for the connection of the main body of the prosthesis to a coupling portion 60.

According to a further embodiment of the present invention, said coupling portion 60 comprises a cup 28 adapted to create kinematic coupling with a glenosphere 36 associated with the corresponding scapula.

According to an advantageous embodiment of the present invention, the main body of the prosthesis 16 is associated, by means of a male-female type form coupling to a coupling portion 60 in the form of a cup 28 adapted to interacting with a glenosphere 36 attachable to a corresponding glenoid 64.

According to one embodiment, the main body of the prosthesis 16 comprises a first connecting element 76, for example parallel to said prevalent extension Y, and the coupling portion 60 comprises a second connecting element 78 having the counter form of said first connecting element 76.

Advantageously, the coupling between the main body of the prosthesis 16 and the cup 28 is an axial type according to a coupling direction parallel to said first and second connecting element 76,78 in a manner to permit the fastening and release of the cup 28 from the main body of the prosthesis 16 in a same axial coupling direction, preferably parallel to said prevalent extension Y.

Advantageously, in an insertion configuration of the cup 28 into the main body of the prosthesis 16, a lower portion 82 of the cup 28, facing the main body of the prosthesis 16, results as aligned with a lower base 84 of said first connecting element 76, said lower base 84 being in a position facing the main body of the prosthesis 16.

According to one embodiment of the present invention, the first connecting element 76 presents a disc 56 having on its side surface, at least one ribbing 68 and said second connecting element 78 presents at least one groove 72 having the counter shape of said ribbing 68.

According to one embodiment of the present invention, said disc 56 presents four ribbings 68 positioned perpendicular to each other and said second connecting element 78 presents four grooves positioned perpendicular to each other to permit coupling with the ribbings.

According to one embodiment, the cup 28 comprises a wedge-shaped portion 88 in order to direct the cup 28 in a direction at an incidence with the glenosphere 36 of the attachable scapula.

Preferably, the area of the section main body of the prosthesis 16 in relation to a plane perpendicular to said prevalent extension Y does not exceed 85% of the total area of a disc 56 associated with the main body of the prosthesis 16 for the connection of the main body of the prosthesis 16 to a coupling portion 60, such as a cup 28.

According to a further embodiment, the first connecting element 76, for example set parallel to said prevalent extension Y, comprises a pocket 77 and the coupling portion 60, in the form of a cup 28, comprises a second connecting element 78 having the counter form of said first connecting element 76. For example, the second connecting element 78 is equipped with an annular ridge 79 suitable for insertion into said pocket 77 by means of form coupling.

Preferably, the main body of the prosthesis 16 is made of a metallic material and the cup 28 is made of a polymer material, preferably very high-density polyethylene (UHDPE). Alternatively, the cup 28 can be made of metallic material. Advantageously, the cup 28 presents a pass-through hole 80, which crosses the body 16 completely in a manner to permit the insertion of a connecting screw 81 between the main body of the prosthesis 16 and the stem 12. The connecting screw 81 presents a threaded section adapted to engagement with the threaded hole 66 of the stem 12. This makes it possible to change from the direct configuration to the inverted configuration of the prosthesis 4 by extracting the main body of the prosthesis 16, previously inserted in the humerus and mounted with a spherical head 24 according to the direct configuration concept, and by inserting a different main body of the prosthesis 16 mounted with a cup 28. Said main body of the prosthesis 16 is preferably circular and free of any fins 40,46.

In particular, the main body of the prosthesis 28 mounted with a cup can be anchored to the stem 12 which has remained inserted into the humerus by means of the insertion of the connecting screw. The removal of the main body of the prosthesis 16 from the stem is made easier by the connection between the main body of the prosthesis 16 and the stem 12 by means of a "morse" cone coupling. After the insertion of the main body of the prosthesis in the inverted configuration, in other words, equipped with a first connecting element 76 having a pocket 77, and after the screwing of the relative connecting screw, it is possible to fasten the cup by means of a click-on action to the main body of the prosthesis by fastening the annular ridge 79 in the pocket 77.

According to a further embodiment of the present invention, said prosthesis 4 comprises means of correction 92 for the relative position between the main body of the prosthesis 16 and the coupling portion 60, adapted to allowing a relative axial sliding action between the disc and the coupling portion 60 along at least one axial direction belonging to a plane perpendicular to said prevalent extension.

According to one embodiment, the means of correction comprises a first distancer 94 adapted to creating a form coupling with said disc 56, and a second distancer 96 adapted to creating a form coupling with the coupling portion 60, said first and second distancers 94,96 being able to move along at least one axial direction belonging to a plane perpendicular to said prevalent extension Y of the main body of the prosthesis 16.

According to one embodiment, at least one of said first and second distancers 94,96 comprises a slide shoe 98 adapted to allowing relative sliding action between said distancers.

The distancers 94,96 can be reciprocally blocked with each other along the slide by means of a continuous type or a discrete type regulation.

According to a further embodiment, the present invention relates to a shoulder prosthesis 100 adapted to being associated with a glenoid 64. The shoulder prosthesis 100 comprises a coupling element 104 suitable for attachment to a glenoid 64 to create a kinematic coupling with an attachable humerus and fixing means 108 of the coupling element 104 to the glenoid 64.

According to one embodiment, the fixing means 108 comprises an expanding element 110 suitable for insertion into a bone in a non-expanded configuration inside the bone in order to provide at least an undercut adapted to preventing the exit of the coupling element 104 from the bone.

The expanding element 110 comprises a portion for insertion 114, suitable for association with the coupling element 104, on the opposite side of the glenoid, and an expanding portion 116.

Preferably, the portion for insertion 114 forms an insertion coupling with the collar 120 of said coupling element 104.

The collar 120 and the portion for insertion 114 form a pass-through cavity 122.

Preferably, the prosthesis 100 comprises a pin 126 suitable for insertion into said cavity 122 through the collar 120 and the portion for insertion 114, in a manner so that it penetrates the expanding portion 116 and provokes expansion.

The expanding portion 116 comprises at least one tang 130 adapted to bending during the change from a non-expanded configuration to an expanded configuration.

Preferably, the expanding portion 116 comprises a plurality of tangs 130 that radially encircle a lower opening 134 in relation to the cavity 122 or in any case, to the diameter of the pin 126 in such a manner that after the insertion of a pin 126, this will interact with said tangs 130 provoking the expansion towards the exterior.

The pin 126 presents a beat head 134 adapted to acting as the end of run during the pin 126 insertion on the coupling element from the opposite side of the expanding element.

Preferably, the portion for insertion 114 comprises ledges 138 adapted to stabilising the prosthesis during rotation and flexion. Preferably, said ledges are positioned along an external rim of said collar.

According to one embodiment, the coupling element 104 is a glenosphere 36 adapted to being fixed to a scapula in such a manner that it will couple with a cup 28 operatively connected to a humerus.

According to a further embodiment, the coupling element 104 is a glenoid prosthesis being concave on the side of the attachable humerus and adapted to being associated with a head of a humerus.

According to a further embodiment, the shoulder prosthesis 100, adapted to being associated with a glenoid, comprises a plate 142 adapted to being fixed to a glenoid 64 to create a kinematic coupling with a head of a humerus. The prosthesis 100 comprises a plate element 146 and means for fastening to the glenoid 150. Preferably, the fastening means 150 comprises at least a catch 154 adapted to fastening onto a rim of the glenoid.

Preferably, the plate element 146 comprises at least two catches positioned on the opposite sides of the plate element, and even more preferably comprises two pairs of catches positioned on the opposite sides of the plate element.

According to one embodiment, the plate element 146 comprises a protuberance 156 adapted to being inserted at least partially into the glenoid 64.

The plate element 146 presents a coupling hole 158 adapted to allowing the insertion onto the plate element 146 of a coupling interface 160 to a humerus.

For example, said coupling interface 160 can be a coating plate 164 adapted to covering the plate element 146 on the opposite side from the glenoid, said plate 164 being concave on the side of the attachable humerus.

The coating plate 164 presents a ridge 168 adapted to being inserted and fixed in said coupling hole 158 of the plate element 146.

According to a further embodiment, said coupling interface 160 is a glenosphere 36 that is convex on the side of the attachable humerus.

According to a further embodiment of the present invention, the shoulder prosthesis 100 comprises a coupling element 104 in the form of a glenosphere 36, associated with the plate 142 adapted to being fixed to a glenoid 64 to create a kinematic coupling with a head of humerus. The plate 142 is produced in a single piece with an attachment protuberance 170 adapted to being inserted into the glenoid 64, preferably having at least a flared or bevelled form 172 to prevent relative rotation between the coupling element 104 and the attachable glenoid 64. Preferably, the fixing protuberance 170 comprises four flared elements 172; preferably at least one flared element having a pass-through hole adapted to favouring the osteointegration of the prosthesis.

Preferably, the prosthesis comprises said cavity 122 adapted to housing the pin 126 to fasten the glenosphere 36 to plate 142 and to the attaching protuberance 170 . The plate 142 preferably presents holes or slots 45 for the possible insertion of fixing pins or to facilitate osteointegration.

As can be appreciated from the aforesaid description, the prosthesis of the present invention is able to overcome all the drawbacks present in prostheses of prior art.

In particular, the prosthesis according to the invention is especially non invasive, because of the reduced volume of the main body of the prosthesis.

The prosthesis according to the present invention provides far easier and optimal results in operations for resuturing the tuberosities of the humeral head (trochite and trochine) with the consequential improvement in articular function recovery. In fact the fins of the main body of the prosthesis create an advantageous support for the tuberosities during the resuturing stage, and as well as making resuturing operations easier for the surgeon, they also ensure correct support for the bone.

The prosthesis according to the present invention allows to correct certain important geometrical parameters such as anterior and posterior and central-side offset in the head-acromion distance without the need for explanting the prosthesis in question.

The prosthesis according to the present invention provides the possibility of post-operatory correction following error or for corrective purposes.

Thanks to the fact that the connecting section of the cup to the main body of the prosthesis is positioned above the anatomic neck of the humerus, this limits further invasive interventions of the prosthesis during any change from direct configuration to inverted configuration.

The prostheses according to the present invention provide safe attachment to the bone without being particularly invasive, and especially in relation to the glenoid. In fact, the use of an expanding element allows a single hole to be realised thus resulting far less invasive compared to the plurality of holes of the screw systems used in prior art.

The drilling of a single hole also limits the danger of drilling errors or ill-positioned holes, as well as the risk of bone fracture in proximity to the hole.

The anchoring system of the prosthesis to the glenoid by means of click-in fins provides the possibility of avoiding hole drilling in the bone; this provides a non-invasive method and yet guarantees very high stability. The elasticity of the fins also permits the correction of positioning errors or in any case, the removal of the prosthesis during implanting without the need for drilling.

The use of a stem without any ribbing permits the humerus to be inserted without damaging the humerus spongy tissue and furthermore, also allows any correction of the angular orientation of the stem and of the main body of the prosthesis that may be necessary without damaging the spongy tissue.

Therefore the use of a stem without ribbing contributes towards limiting invasive intervention of the prosthesis.

Advantageously, the area of the sections of the main body of the prosthesis, such as the fins for example, does not exceed 85% of the total area of the connecting element of the main body of the prosthesis to the coupling portion. In this manner the connecting element, having the form of a disc for example, resists against the penetration of the prosthesis into the humerus to ensure that the connecting element always remains in a position that is not lower than the neck of the humeral head.

In order to satisfy pertinent and specific necessities, those skilled in the art will be able to apply numerous modifications and variants to the aforesaid prosthesis, while remaining within the scope of the invention as defined in the following claims.

## Claims

1. Shoulder prosthesis (4) comprising
- a stem (12) suitable for being inserted into a humerus (6) for the attachment of the prosthesis on the humerus (6), the stem (12) forming a direction of insertion (X),
- a main body of the prosthesis (16), attachable to said stem (12), and suitable for being inserted at least partially into a humerus (6) positioned on a humeral head (8) presenting a pair of tuberosities (20),
- the main body (16) of the prosthesis extending according to a prevalent extension (Y) set at an angle in relation to the insertion direction (X) in a such a manner to follow the anatomic angle of the humeral head (8) and to provide an internal curved part and an external curved part of said main body (16) of the prosthesis with respect to said axis of the prevalent extension (Y),
- the main body (16) of the prosthesis being adapted to be associated with a spherical head (24) or with a cup (28) in a manner so that it interacts with a glenoid cavity (32) or with a glenosphere (36) respectively,
**characterized in that**
the main body (16) of the prosthesis comprises two support fins (40) that extend radially with respect to said axis of prevalent extension (Y), said two support fins (40) being arranged and defining said internal curved part of said main body (16) of the prosthesis said two support fins (40) forming between them, in relation to a plane perpendicular to said prevalent extension (Y), a flare angle (α) between 60 and 120 degrees in a manner to be abutted by the tuberosities (20) of the humerus (6) in an insertion configuration of the prosthesis in the humerus (6), said two support fins (40) having a blade-like configuration and extending from said axis of prevalent extension (Y) in a manner so that the main body (16) of the prosthesis assumes a section that is bilobate;
wherein said main body (16) of the prosthesis presents a third fin (46) symmetrically arranged in relation to said two support fins (40), said third fin (46) defining said external curved part of said main body (16) of the prosthesis and extending from the side opposite the two support fins (40) in relation to the axis of prevalent extension (Y) in a manner so that the main body (16) of the prosthesis assumes an overall "Y"-shaped section.

2. Shoulder prosthesis (4) according to claim 1, wherein said fins (40) that together form, in relation to a plane perpendicular to said set at an angle direction, a flare angle (α) between 80 and 100 degrees.

3. Shoulder prosthesis (4) according to claim 1 or 2. wherein said fins (40) that together form, in relation to a plane perpendicular to said set at an angle direction, a flare angle (α) equal to 90 degrees.

4. Shoulder prosthesis (4) according to any one of the previous claims, wherein said fins (40) present at least one hole (44) or slot (45) adapted to allowing the passage of the suture for the resuturing of said tuberosities (20).

5. Shoulder prosthesis (4) according to any one of the previous claims, wherein at least one of said fins (40,46), extends in relation to the axis of prevalent extension (Y) for an increasing distance moving from a first end (50) of the main body (16) of the prosthesis on the side of the attachable stem (12) to a second end (52) of the main body (16) of the prosthesis opposite to said first end (50).

6. Shoulder prosthesis (4) according to any one or the previous claims, wherein said fins (40, 46), on the side opposite the stem (12) are operatively connected to a detachable disc (56) by means of form coupling to a coupling portion (60) adapted to creating a kinematic coupling with the scapula.

7. Shoulder prosthesis (4) according to claim 6, wherein said coupling portion (60) comprises a spherical head (24), on the opposite of the main body (16) of the prosthesis adapted to creating a kinematic coupling with a glenoid (64) of the attachable scapula.

8. Shoulder prosthesis (4) according to claim 6 or 7, wherein said disc (56) presents at least one ribbing (68) positioned on a shim and said coupling portion (60) presents at least a groove (72) having the counter form of said ribbing (68).

9. Shoulder prosthesis (4) according to any one of the claims from 6 to 8, wherein said coupling portion (60) comprises a cup (28) adapted to creating kinematic coupling with a glenosphere (36) associated with the corresponding scapule.

10. Shoulder prosthesis (4) according to any one of the previous claims, wherein said stem (12) has an axis of symmetry in relation lo said insertion direction, in a manner to provide a revolving surface adapted to rotation inside the said humerus to permit the correct angular positioning of the prosthesis therein.

11. Shoulder prosthesis (4) according to any one of the previous claims, wherein the area of the fin section (40,46) in relation to the plane perpendicular to said prevalent extension (Y) does not exceed 85% of the total area of the disc (56) associated with the main (16) of the prosthesis for the connection of the main body (16) of the prosthesis to a coupling portion (60).

12. Shoulder prosthesis (4) according to any one of the previous claims, wherein the main (16) of the prosthesis comprises at a first end (50) a first cone (57), of the 'morse' type adapted to favouring the attachment of a coupling portion (60) of the prosthesis (4), such as a spherical head (24) or a cup (28), to the main body (16) of the prosthesis by means of form coupling.

13. Shoulder prosthesis (4) according to claim 12, wherein the coupling portion (60, 24, 28) comprises a spherical head (24) having a housing in the snape of the counter form of the first cone (57) in a manner to ensure the secure blocking of the spherical head (24) on the main body (16) of the prosthesis.

14. Shoulder prosthesis (4) according to any one of the previous claims, wherein the connection between the main body (16) of the prosthesis and the stem (12), at one end (50) of the main body (16) of the prosthesis is a form coupling.

## Patentansprüche

1. Schulterprothese (4), umfassend:
- einen Stamm (12), der dazu geeignet ist, in einen Humerus (6) eingefügt zu werden, für die Befestigung von der Prothese an dem Humerus (6), wobei der Stamm (12) eine Einfügungsrichtung (X) bildet,
- einen Hauptkörper von der Prothese (16), der an dem Stamm (12) befestigbar und dazu geeignet ist, zumindest teilweise in einen Humerus (6) eingefügt zu werden, positioniert an einem Humeruskopf (8), der ein Paar von Tuberositae (20) aufweist,
- der Hauptkörper (16) von der Prothese erstreckt sich gemäß einer vorherrschenden Erstreckung (Y), die in einem Winkel bezogen auf die Einfügungsrichtung (X) derart festgelegt ist, dass sie dem anatomischen Winkel von dem Humeruskopf (8) folgt und einen internen gekrümmten Teil und einen externen gekrümmten Teil von dem Hauptkörper (16) von der Prothese bezogen auf die Achse von der vorherrschenden Erstreckung (Y) bereitstellt,
- der Hauptkörper (16) von der Prothese ist dazu angepasst, mit einem sphärischen Kopf (24) oder mit einer Schale (28) derart verbunden zu werden, dass er mit einer Schulterpfanne (32) beziehungsweise mit einer Glenosphäre (36) interagiert,
**dadurch gekennzeichnet, dass**
der Hauptkörper (16) der Prothese zwei Stützgrate (40) umfasst, die sich bezogen auf die Achse von der vorherrschenden Erstreckung (Y) radial erstrecken, wobei die zwei Stützgrate (40) angeordnet sind und den internen gekrümmten Teil des Hauptkörpers (16) der Prothese definieren,
die zwei Stützgrate (40) bilden zwischen ihnen, bezogen auf eine Ebene senkrecht zu der vorherrschenden Erstreckung (Y), einen Öffnungswinkel (α) zwischen 60 und 120 Grad, dergestalt, dass sich die Tuberositae (20) von dem Humerus (6) in einer Einfügungskonfiguration von der Prothese in dem Humerus (6) daran angrenzen, wobei die zwei Stützgrate (40) eine blattähnliche Konfiguration haben und sich von der Achse von der vorherrschenden Erstreckung (Y) derart erstrecken, dass der Hauptkörper (16) von der Prothese einen Schnitt annimmt, der zweilappig ist;
wobei der Hauptkörper (16) von der Prothese einen dritten Grat (46) aufweist, der symmetrisch zwischen den zwei Stützgraten (40) angeordnet ist, wobei der dritte Grat (46) den besagten externen gekrümmten Teil von dem besagten Hauptkörper (16) von der Prothese definiert und sich von der Seite gegenüber den zwei Stützgraten (40) bezogen auf die Achse von der vorherrschenden Erstreckung (Y) derart erstreckt, dass der Hauptkörper (16) von der Prothese einen insgesamt "Y"-förmigen Schnitt annimmt.

2. Schulterprothese (4) nach Anspruch 1, wobei die besagten Grate (40), die zusammen, bezogen auf eine Ebene senkrecht zu der besagten in einer Winkelrichtung festgelegten, einen Öffnungswinkel (α) zwischen 80 und 100 Grad bilden.

3. Schulterprothese (4) nach Anspruch 1 oder 2, wobei die besagten Grate (40), die zusammen, bezogen auf eine Ebene senkrecht zu der besagten in einer Winkelrichtung festgelegten, einen Öffnungswinkel (α) gleich 90 Grad bilden.

4. Schulterprothese (4) nach einem der vorhergehenden Ansprüche, wobei die besagten Grate (40) mindestens ein Loch (44) oder einen Schlitz (45) aufweisen, das/der dazu angepasst ist, den Durchgang von dem Faden für das erneute Zunähen von den besagten Tuberositae (20) zu erlauben.

5. Schulterprothese (4) nach einem der vorhergehenden Ansprüche, wobei sich mindestens einer der besagten Grate (40, 46) bezogen auf die Achse von der vorherrschenden Erstreckung (Y) für eine zunehmende Distanz, sich von einem ersten Ende (50) von dem Hauptkörper (16) von der Prothese auf der Seite von dem befestigbaren Stamm (12) zu einem zweiten Ende (52) von dem Hauptkörper (16) der Prothese gegenüber dem besagten ersten Ende (50) bewegend, erstreckt.

6. Schulterprothese (4) nach einem der vorhergehenden Ansprüche, wobei die besagten Grate (40, 46) auf der Seite gegenüber des Stamms (12) mittels Formkupplung an einen Kupplungsabschnitt (60), der dazu angepasst ist, eine kinematische Kupplung mit dem Schulterblatt zu erschaffen, mit einer lösbaren Platte (56) operativ verbunden sind.

7. Schulterprothese (4) nach Anspruch 6, wobei der Kupplungsabschnitt (60) einen sphärischen Kopf (24) gegenüber dem Hauptkörper (16) von der Prothese umfasst, der dazu angepasst ist, eine kinematische Kupplung mit einer Gelenkpfanne (64) von dem befestigbaren Schulterblatt zu erschaffen.

8. Schulterprothese (4) nach Anspruch 6 oder 7, wobei die Platte (56) zumindest eine Rippung (68) aufweist, die auf einer Scheibe positioniert ist, und der Kupplungsabschnitt (60) mindestens eine Nut (72) mit der Gegenform von der besagten Rippung (68) aufweist.

9. Schulterprothese (4) nach einem der Ansprüche 6 bis 8, wobei der Kupplungsabschnitt (60) eine Schale (28) umfasst, die dazu angepasst ist, eine kinematische Kupplung mit einer Glenosphäre (36), die mit dem korrespondierenden Schulterblatt verbunden ist, zu erschaffen.

10. Schulterprothese (4) nach einem der vorhergehenden Ansprüche, wobei der Stamm (12) eine Symmetrieachse bezogen auf die Einfügungsrichtung hat, in einer Weise, um eine drehbar gelagerte Fläche bereitzustellen, die für eine Drehung in den besagten Humerus hinein angepasst ist, um die korrekte Winkelpositionierung von der Prothese darin zu ermöglichen.

11. Schulterprothese (4) nach einem der vorhergehenden Ansprüche, wobei der Bereich von dem Gratabschnitt (40, 46) bezogen auf die Ebene senkrecht zu der besagten vorherrschenden Erstreckung (Y) 85% von dem Gesamtbereich von der Platte (56) verbunden mit dem Hauptteil (16) von der Prothese für die Verbindung von dem Hauptkörper (16) von der Prothese mit einem Kupplungsabschnitt (60) nicht überschreitet.

12. Schulterprothese (4) nach einem der vorhergehenden Ansprüche, wobei der Hauptteil (16) von der Prothese an einem ersten Ende (50) einen ersten Konus (57) von dem "Morse"-Typ umfasst, der dazu angepasst ist, die Befestigung von dem Kupplungsabschnitt (60) von der Prothese (4), wie etwa ein sphärischer Kopf (24) oder eine Schale (28), an dem Hauptkörper (16) von der Prothese mittels Formkupplung zu begünstigen.

13. Schulterprothese (4) nach Anspruch 12, wobei der Kupplungsabschnitt (60, 24, 28) einen sphärischen Kopf (24) mit einem Gehäuse in dem "Snape" von der Gegenform von dem ersten Konus (57) umfasst, in einer Weise, um das sichere Blockieren von dem sphärischen Kopf (24) an dem Hauptkörper (16) von der Prothese sicherzustellen.

14. Schulterprothese (4) nach einem der vorhergehenden Ansprüche, wobei die Verbindung zwischen dem Hauptkörper (16) von der Prothese und dem Stamm (12) an einem Ende (50) von dem Hauptkörper (16) von der Prothese eine Formkupplung ist.

## Revendications

1. Prothèse d'épaule (4) comprenant
- une tige (12) adaptée pour être insérée dans un humérus (6) pour la fixation de la prothèse sur l'humérus (6), la tige (12) formant une direction d'insertion (X),
- un corps principal de la prothèse (16), pouvant être fixé à ladite tige (12), et adapté pour être inséré au moins partiellement dans un humérus (6) positionné sur une tête humérale (8) présentant une paire de tubérosités (20),
- le corps principal (16) de la prothèse s'étendant selon une extension prévalente (Y) réglée à un angle par rapport à la direction d'insertion (X) de manière à suivre l'angle anatomique de la tête humérale (8) et à fournir une partie incurvée interne et une partie incurvée externe dudit corps principal (16) de la prothèse par rapport audit axe de l'extension prévalente (Y),
- le corps principal (16) de la prothèse étant adapté pour être associé à une tête sphérique (24) ou à une cuvette (28) de manière à interagir avec une cavité glénoïde (32) ou avec une glénosphère (36) respectivement,
**caractérisée en ce que**
le corps principal (16) de la prothèse comprend deux ailettes de support (40) qui s'étendent radialement par rapport audit axe d'extension prévalente (Y), lesdites deux ailettes de support (40) étant agencées et définissant ladite partie incurvée interne dudit corps principal (16) de la prothèse,
lesdites deux ailettes de support (40) formant entre elles, par rapport à un plan perpendiculaire à ladite extension prévalente (Y), un angle d'évasement (α) entre 60 et 120 degrés de manière à être aboutées par les tubérosités (20) de l'humérus (6) dans une configuration d'insertion de la prothèse dans l'humérus (6), lesdites deux ailettes de support (40) ayant une configuration semblable à une lame et s'étendant dudit axe d'extension prévalente (Y) de manière à ce que le corps principal (16) de la prothèse adopte une section qui est bilobée ;
dans laquelle ledit corps principal (16) de la prothèse présente une troisième ailette (46) agencée symétriquement par rapport auxdites deux ailettes de support (40), ladite troisième ailette (46) définissant ladite partie incurvée externe dudit corps principal (16) de la prothèse et s'étendant du côté opposé aux deux ailettes de support (40) par rapport à l'axe d'extension prévalente (Y) de manière à ce que le corps principal (16) de la prothèse adopte une section en forme de « Y » dans l'ensemble.

2. Prothèse d'épaule (4) selon la revendication 1, dans laquelle lesdites ailettes (40) qui forment ensemble, par rapport à un plan perpendiculaire audit ensemble à une direction d'angle, un angle d'évasement (α) entre 80 et 100 degrés.

3. Prothèse d'épaule (4) selon la revendication 1 ou 2, dans laquelle lesdites ailettes (40) qui forment ensemble, par rapport à un plan perpendiculaire audit ensemble à une direction d'angle, un angle d'évasement (α) égal à 90 degrés.

4. Prothèse d'épaule (4) selon l'une quelconque des revendications précédentes, dans laquelle lesdites ailettes (40) présentent au moins un trou (44) ou une fente (45) adapté pour permettre le passage de la suture pour resuturer lesdites tubérosités (20).

5. Prothèse d'épaule (4) selon l'une quelconque des revendications précédentes, dans laquelle au moins une desdites ailettes (40, 46), s'étend par rapport à l'axe d'extension prévalente (Y) sur une distance croissante allant d'une première extrémité (50) du corps principal (16) de la prothèse sur le côté de la tige pouvant être fixée (12) à une deuxième extrémité (52) du corps principal (16) de la prothèse opposée à ladite première extrémité (50).

6. Prothèse d'épaule (4) selon l'une quelconque des revendications précédentes, dans laquelle lesdites ailettes (40, 46), sur le côté opposé à la tige (12) sont reliées de manière fonctionnelle à un disque amovible (56) par couplage de forme à une portion de couplage (60) adaptée pour créer un couplage cinématique avec l'omoplate.

7. Prothèse d'épaule (4) selon la revendication 6, dans laquelle ladite portion de couplage (60) comprend une tête sphérique (24), sur le côté opposé du corps principal (16) de la prothèse, adaptée pour créer un couplage cinématique avec un glénoïde (64) de l'omoplate pouvant être fixée.

8. Prothèse d'épaule (4) selon la revendication 6 ou 7, dans laquelle ledit disque (56) présente au moins une nervure (68) positionnée sur une cale et ladite portion de couplage (60) présente au moins une rainure (72) ayant la contreforme de ladite nervure (68).

9. Prothèse d'épaule (4) selon l'une quelconque des revendications 6 à 8, dans laquelle ladite portion de couplage (60) comprend une cuvette (28) adaptée pour créer un couplage cinématique avec une glénosphère (36) associée à l'omoplate correspondante.

10. Prothèse d'épaule (4) selon l'une quelconque des revendications précédentes, dans laquelle ladite tige (12) a un axe de symétrie par rapport à ladite direction d'insertion, de manière à fournir une surface tournante adaptée à la rotation à l'intérieur dudit humérus pour permettre le positionnement angulaire correct de la prothèse à l'intérieur de celui-ci.

11. Prothèse d'épaule (4) selon l'une quelconque des revendications précédentes, dans laquelle ladite surface de la section d'ailette (40, 46) par rapport au plan perpendiculaire à ladite extension prévalente (Y) ne dépasse pas 85 % de la surface totale du disque (56) associé au principal (16) de la prothèse pour la connexion du corps principal (16) de la prothèse à une portion de couplage (60).

12. Prothèse d'épaule (4) selon l'une quelconque des revendications précédentes, dans laquelle le principal (16) de la prothèse comprend à une première extrémité (50) un premier cône (57), du type « morse » adapté pour favoriser la fixation d'une portion de couplage (60) de la prothèse (4), comme une tête sphérique (24) ou une cuvette (28), au corps principal (16) de la prothèse, par couplage de forme.

13. Prothèse d'épaule (4) selon la revendication 12, dans laquelle la portion de couplage (60, 24, 28) comprend une tête sphérique (24) ayant un logement dans la forme de la contreforme du premier cône (57) de manière à assurer le blocage sûr de la tête sphérique (24) sur le corps principal (16) de la prothèse.

14. Prothèse d'épaule (4) selon l'une quelconque des revendications précédentes, dans laquelle la connexion entre le corps principal (16) de la prothèse et la tige (12), à une extrémité (50) du corps principal (16) de la prothèse est un couplage de forme.
